# EUROPEAN PATENT APPLICATION

(11) **EP 0 702 086 A1**
(43) Date of publication of application: **20.03.1996**
(21) Application number: 95113582.1
(22) Date of filing: 30.08.1995
(51) Int. Cl.: C12P 17/04, C12P 17/02

(54) **Process for producing antibiotics**

(30) Priority: 14.09.1994 US 305625
(71) Applicant: F. HOFFMANN-LA ROCHE AG, CH-4002 Basel (CH)
(72) Inventor: Block, David Eric, Lawrenceville, N.J. 08648 (US); Hermann, Theron Edwin, Kinnelon, N.J. 07405 (US); Hsieh, Jih-Han, Parsippany, N.J. 07054 (US); Mehta, Nikhil Sureshchandra, Livingston, N.J. 07039 (US); Rai, Vishva Roop, Randolph, N.J. 07869 (US)
(74) Representative: Kellenberger, Marcus, Dr.

(57) **Abstract**

A process for producing deoxyfrenolicin and optionally frenolicin B comprises fermenting a broth containing a microorganism capable of producing frenolicin under aerobic conditions to produce frenolicin, then converting the frenolicin in the same broth under anaerobic conditions to deoxyfrenolicin, and if desired converting the deoxyfrenolicin to frenolicin B during the subsequent recovery and purification steps. Each of the three frenolicins is known to be useful as an antibiotic, especially for administration to animals.

## Description

The invention relates to the novel production of known antibiotics, in particular of deoxyfrenolicin from frenolicin and of frenolicin B from the intermediate deoxyfrenolicin.

The biosynthesis of frenolicin, a compound of the formula 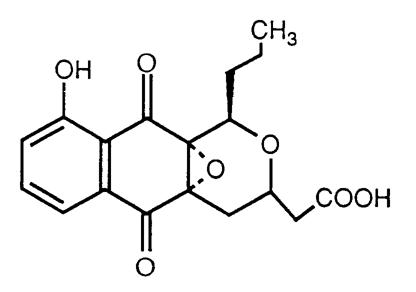
with antibiotic activity, was first reported in Antimicrob. Ag. Ann. 1960, 77-80 (1961). Frenolicin was produced by cultivation of a soil isolate identified as *Streptomyces fradiae* under conventional agitation and aeration conditions.

It was later reported that frenolicin could be chemically converted to deoxyfrenolicin, a compound of the formula 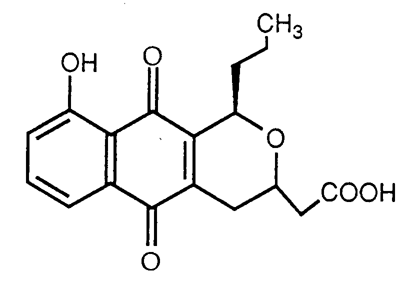
by reduction with any of a variety of agents [see J.A.C.S. 88, 4109 (1966); J.A.C.S 90, 1325 (1968); and U.S Patent Specification 3,452,051].

The biosynthesis of deoxyfrenolicin was first reported in U.S. Patent Specification 4,199,514 and J. Antibiot. 31, 959-965 (1978). These documents describe the fermentation of a broth containing the *Streptomyces roseofulvus* strain AM-3867 (FERM-P No. 4359 and ATCC No. 31476) under aeration and aerobic conditions. Such fermentation produces a culture broth containing frenolicin, deoxyfrenolicin and a further antibiotic, frenolicin B, of the formula 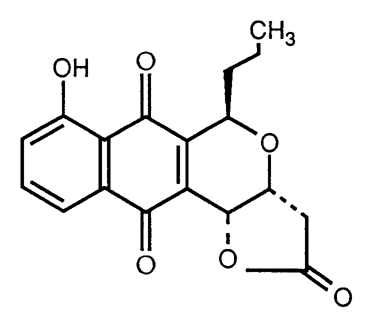
After completion of the fermentation, the culture broth is separated into the bacterial cells and the filtrate. The filtrate of a culture broth produced by fermentation of a *Streptomyces* microorganism can be treated by conventional methods, such as by changing the pH or temperature (see Belter et al., Bioseparations - Downstream Processing for Biotechnology, J. Wiley & Sons, New York 1988, pp. 17, 25, 99 and 105). However, previous procedures to separate and recover the antibiotics after completion of the fermentation described in U.S. Patent Specification 4,199,514 and J. Antibiot. 31, 959-965 (1978) resulted in a recovery yield of frenolicin B of less than 50%.

In essence, the present invention is directed to a process for producing deoxyfrenolicin, and if desired thereafter, frenolicin B. The process comprises fermenting a broth (nutrient medium) under aerobic conditions so as to produce frenolicin, said broth containing a microorganism capable of producing frenolicin. The frenolicin is then converted in the same broth (containing the microorganism) under anaerobic conditions to deoxyfrenolicin. If desired, the deoxyfrenolicin is converted to frenolicin B during the subsequent recovery and purification steps.

It has surprisingly been discovered that, after the fermentation has been effected to produce frenolicin, the conversion of this to deoxyfrenolicin in the absence of oxygen (anaerobic conditions) and in the presence of the previously used microorganism (bacterial) cells in the same broth improves the ultimate yield of frenolicin B over that obtained previously. Furthermore, this procedure saves processing time, raw material and equipment, and also simplifies the recovery step. In particular, it enables the same fermentation equipment for producing frenolicin to be used for converting frenolicin to deoxyfrenolicin.

Accordingly, the present invention concerns a process for producing deoxyfrenolicin, and if desired, frenolicin B, which process is characterized by a) fermenting a broth containing a microorganism capable of producing frenolicin under aerobic conditions to produce frenolicin, then b) converting the frenolicin in the same broth under anaerobic conditions to deoxyfrenolicin, and if desired, c) converting the deoxyfrenolicin to frenolicin B during the subsequent recovery and purification steps.

In the present invention, any microorganism may be used that is capable of producing the compound frenolicin. Preferred are mutant strains of *Streptomyces roseofulvus* obtained by subjecting *Streptomyces roseofulvus* to mutagenic treatment. A preferred mutant strain, *Streptomyces roseofulvus* AM-3867, is more specifically described in U.S. Patent Specification 4,199,514. The most preferred mutant strain is the *Streptomyces roseofulvus* mutant strain deposited with the American Type Culture Collection (ATCC), Maryland U.S.A. under the ATCC No. 55598 on July 15, 1994. Other microorganisms which may be used in the invention include *Streptomyces fradiae* [Antimicrob. Ag. Ann. 1960, 77-80 (1961), ATCC No. 10745] and the mutant strain of *Streptomyces roseofulvus* deposited with the ATCC under the No. 19921.

The preparation of the microorganism and the actual fermentation to frenolicin may be carried out in any conventional manner. For example, the microorganism is maintained as lyophilized stock or frozen in liquid nitrogen with 5% lactose and 10% glycerol. The lyophilized stock may be thawed and rehydrated with sterilized deionized water. The culture may be serially diluted and spread on agar slants and/or plates to grow and produce spores in an incubator. The spores are scraped off from the slants or plates to inoculate a seed medium in Erlenmeyer flasks or frozen in 5% lactose and 10% glycerol for later inoculations. The seed medium comprises, for example, starch, dextrose, Bacto tryptone, yeast extract and calcium carbonate. After incubation of the inoculated seed medium, for example in a rotary shaker, the grown microorganism is inoculated into a nutrient medium for fermentation. A typical nutrient medium for producing the frenolicin in the first step (a) of the process according to the present invention contains corn oil, dextrin, corn steep solids, cane molasses, sodium formate or citrate, yeast extract, soya flour, magnesium sulphate, monobasic sodium phosphate, calcium carbonate and an antifoam agent such as polypropyleneglycol monobutyl ether.

The fermentation temperature is generally from about 25°C to about 34°C. The fermentation is completed after a suitable time, when the titer (concentration) of frenolicin is judged to have reached its maximum, i.e. when the rate of production of frenolicin is no longer increasing over time. The titer of frenolicin may be determined, for example, by a HPLC assay method. A typical fermentation time is about 160 to about 240 hours.

In the fermentation broth, there are formed and accumulated frenolicin and a small quantity of deoxyfrenolicin. The subsequent anaerobic treatment of the broth [step b)] promotes the in situ conversion of frenolicin to deoxyfrenolicin. Such treatment preferably comprises stripping the broth of oxygen with nitrogen from the point in time when the titer of frenolicin is at or approximately at its maximum. Upon and while stripping the broth of oxygen, the pH of the broth should be about 7.0 to about 9.0, preferably about 7.5 to about 8.4, and most preferably about 7.9. If necessary, the pH can be adjusted to about 7.9 by addition of an appropriate amount of an acid which is physiologically compatible with the bacterial cells, preferably of phosphoric acid. The broth is then held under anaerobic conditions for about 2 to about 8 hours, depending on the medium composition and frenolicin concentration. After establishment, conveniently by a HPLC assay method, of completion of the conversion, the pH of the broth may be adjusted to about 10 to about 11.5 by addition of an appropriate amount of any base, preferably sodium hydroxide or ammonium hydroxide, and held for an additional 0.5 to 1 hour under anaerobic conditions. The pH adjustment is for inactivating the cellular activity. The temperature of the fermentation broth during the anaerobic treatment is not a critical parameter provided said temperature is not so high as to destroy the microorganism, and temperatures in the range about 15°C to 27°C are conveniently employed. Best results have bean achieved in the upper region of this range.

The preferred process for the conversion to and recovery of frenolicin B is shown in the flow chart represented in Fig. 1 (in which the preferred solvents are indicated).

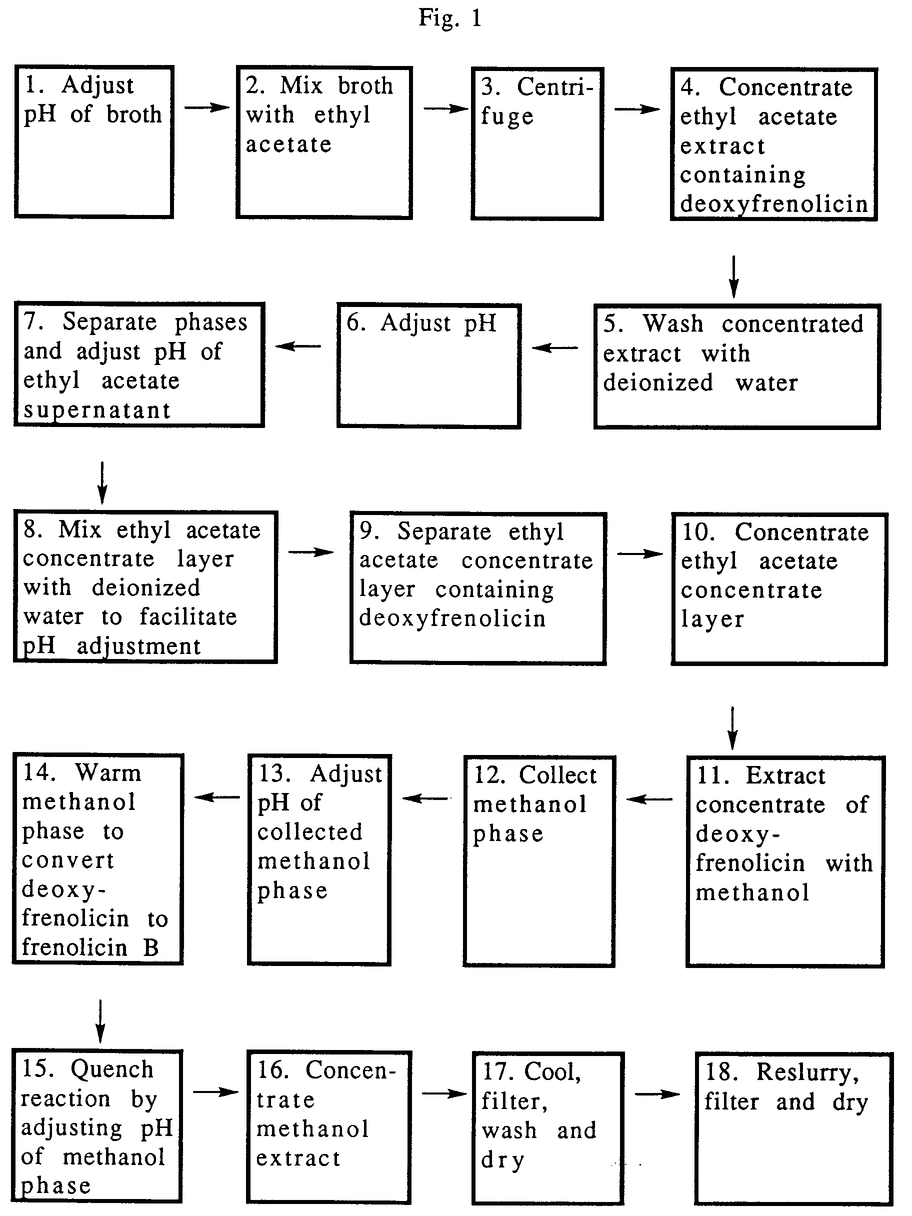

After completion of the conversion, preferably as determined by a HPLC assay method, the resulting deoxyfrenolicin is separated and collected from the fermentation broth. Preferably, the fermentation broth is adjusted to a pH of about 2 to 5.5 by addition of an appropriate amount of any acid, preferably sulphuric or hydrochloric acid (1), and mixed with about 0.5 to about 2 times its volume of a lower alkyl acetate, preferably ethyl acetate, as the solvent at a pH of about 3 (2). The whole fermentation broth and solvent mixture is centrifuged to obtain a solvent, preferably ethyl acetate, supernatant (3). The heavy bottom phase including the extracted broth, cells and solids is separated and discarded. The remaining solvent extract is then concentrated to a deoxyfrenolicin concentration of about 15 to 20 g/l (4).

The collected deoxyfrenolicin is further processed before conversion to frenolicin B in order to remove impurities. Preferably, the above solvent concentrate is washed with about 0.3 to 1 times its volume of deionized water (5). The pH is adjusted to about 5.5 to 6.3, preferably by addition of an appropriate amount of ammonium hydroxide as the base, and the solvent concentrate/water mixture is mixed for about 0.25 to 0.75 hour and allowed to settle for phase separation (6). The resulting solvent concentrate supernatant is separated and its pH adjusted to about 2.8 to 3.5 by addition of an appropriate amount of any acid, preferably sulphuric or hydrochloric acid (7). The solvent concentrate layer is suitably mixed with about 0.12 to 0.17 times its volume of deionized water to facilitate pH adjustment (8). The solvent concentrate layer containing the deoxyfrenolicin is separated from the water layer (9), and the water-washed solvent extract concentrate then further concentrated to an oily paste or until no distillate comes off (10).

The deoxyfrenolicin is then converted during the ensuing recovery and purification steps to frenolicin B, involving warming the deoxyfrenolicin to a temperature up to about 45°C, preferably at an excess pressure above atmospheric pressure of about 0.35 to about 0.70 kg/cm² (atmospheric pressure itself is about 1.033 kg/cm²). The oily paste concentrate is extracted with about 12 to 18 times its volume of a lower alkanol, e.g. methanol or ethanol, preferably the former, depending on the residual oil content in the harvested fermentation broth, to a deoxyfrenolicin concentration of about 7 to about 12 g/l in the alkanol extract (11). The upper alkanol phase is collected (12), its pH adjusted to about 6.0 to 6.2 by addition of an appropriate amount of ammonium hydroxide, and mixed for about 0.2 to 0.5 hours (13). The alkanol extract is then warmed (reacted) for about 9 to about 16 hours at about 36°C to about 45°C and at an air pressure of about 5 to 10 psig [pounds per square inch guage, being the excess pressure above the normal (atmospheric) pressure of 14.7 psi] to convert the deoxyfrenolicin to frenolicin B (14). 5-10 psig is equivalent to about 0.35-0.70 kg/cm². The reaction (conversion) is quenched by adjusting the pH of the alkanol extract to about 2.8 to about 3.4 by addition of an appropriate amount of any acid, preferably sulphuric or hydrochloric acid (15).

The frenolicin B is then crystallized. The alkanol, preferably methanol, extract is concentrated to a frenolicin B concentration of about 45 to about 60 g/l (16). The resulting concentrated slurry is cooled at 0°C to 4°C for about 16 to about 24 hours, the slurry is filtered and the resulting wet cake is washed with an equal volume of cold (for example in the temperature range of about 1-15°C, preferably at about 4°C) lower alkanol, preferably methanol, having a pH of about 2, and is dried (17). The dried frenolicin B solids are conveniently reslurried in about 10 times their volume of a lower (particularly C₅₋₁₀)alkane, preferably n-hexane, for about 10 to about 15 minutes, the resulting slurry is filtered, and the resulting cake dried (18). The alkanol mother liquor can be concentrated to recover a second crop of frenolicin B.

An alternative process for the conversion to and recovery of frenolicin B is shown in the flow chart represented in Fig. 2 (in which the preferred solvents are indicated).

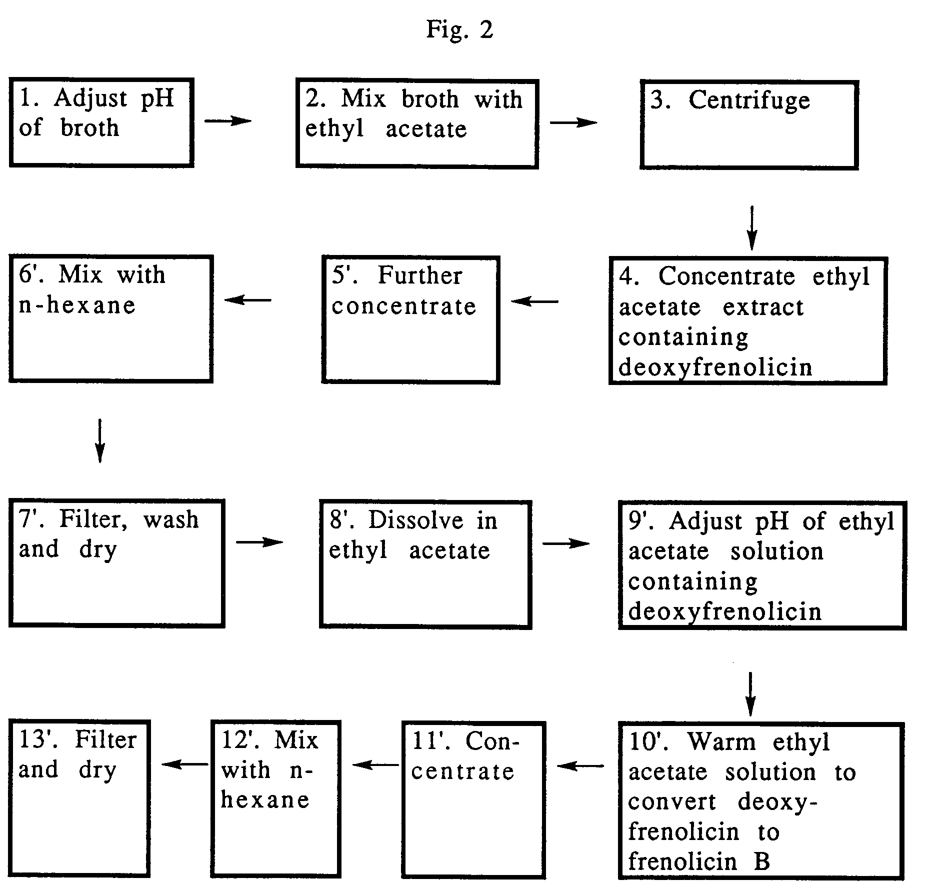

This alternative process for converting the deoxyfrenolicin to frenolicin B eliminates the water washing step and alkanol (preferably methanol) extraction steps of the above described process. This process comprises further concentrating the lower alkyl acetate, preferably ethyl acetate, extract concentrate having a deoxyfrenolicin concentration of about 15 to 20 g/l to an oily paste or until no distillate comes off the concentrate (5'). The oily paste concentrate is mixed with about 1 to 3 times its volume of a lower alkane, preferably n-hexane, for about 0.25 to 0.5 hours to dissolve the oils (6'). The deoxyfrenolicin alkane slurry is filtered, and the resulting cake is washed with about 2 to 3 times its volume of a lower (particularly C₅₋₁₀)alkane, preferably n-hexane, and dried (7'). The solids are redissolved in a lower alkyl, preferably ethyl, acetate to a deoxyfrenolicin concentration of about 30 to about 55 g/l (8'). The pH of the solution is adjusted to about 5.9 to about 6.8 by addition of an appropriate amount of ammonium hydroxide (9'). The lower alkyl acetate solution is warmed (reacted) for about 16 to about 36 hours at about 36°C to about 45°C under an air blanket at about 5 to about 10 psig to convert deoxyfrenolicin to frenolicin B (10'). The converted frenolicin B in the solution is concentrated to a solvent-free frenolicin B slurry (11'). The slurry is mixed with about 1 to about 2 times its volume of a lower alkane, preferably n-hexane (12') and filtered, and the resulting cake is dried (13').

In either process, the dried cake is then preferably recrystallized as shown in the flow chart represented in Fig. 3 (in which the preferred solvent are indicated).

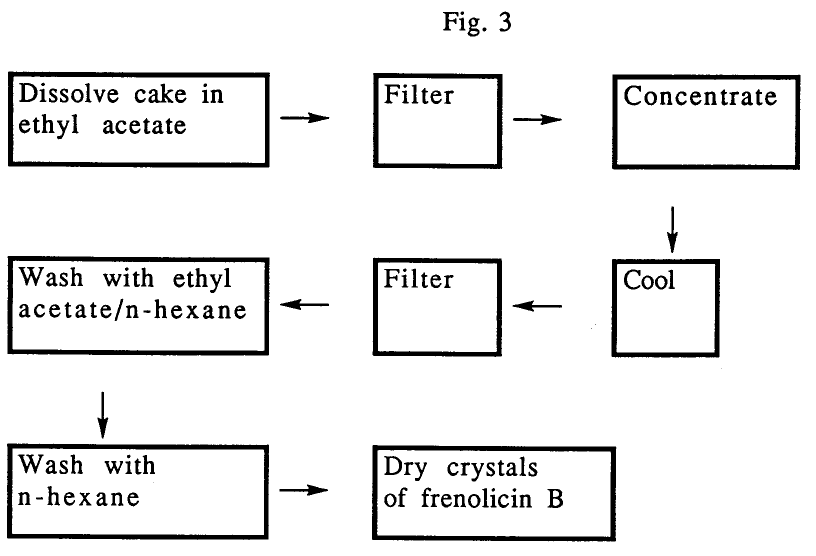

The dried cake is conveniently dissolved in a lower alkyl, preferably ethyl, acetate at ambient temperature to a concentration of from about 70 to about 100 g/l. The solution is filtered and the filtrate is concentrated in nitrogen and under vacuum conveniently in the temperature range of about 25-40°C, preferably at about 35°C, to a frenolicin B concentration of about 275 to 350 g/l. The concentrate is cooled under a filtered nitrogen blanket and the slurry filtered under cold conditions, conveniently in the temperature range of about 1-15°C, preferably at about 4°C. The cake is washed with cold (for example in the aforementioned temperature range, preferably at about 4°C) 25/75 v/v% to 50/50 v/v% lower alkyl acetate/lower alkane solution and then with lower alkane. The mixture of solvents is preferably ethyl acetate/n-hexane, most preferably a 50/50 v/v% mixture of these, and the lower alkane used subsequently is preferably n-hexane. The frenolicin B crystals are then dried.

The following Examples illustrate the invention.

### Examples

### Determination of Frenolicins

In the following Examples of the process of the present invention the following HPLC assay method was used to analyze frenolicin, deoxyfrenolicin and frenolicin B. A Hewlett Packard (HP) HPLC system including HP 1050 series variable wavelength detector, pumping systems and a HP 3396 series II integrator was used with a Waters HPLC column Nova-Pak® C18 (3.9 x 150 mm) with a C18 Guard-PAKS precolumn. The column was operated at ambient temperatures (20 to 25°C) and 1,800 pounds per square inch (psi)/126.5 kg/cm² pressure. The mobile phase solution was a mixture of 60% 2% acetic acid and 40% acetonitrile (v/v) filtered through a 0.2 micron filter and deaerated with helium.

Fermentation broth samples were diluted ten times with acidic methanol (1:500 HCl:methanol) and filtered through a 0.45 micron filter before injection in the HPLC column. Samples taken during the recovery and purification process were diluted with appropriate volumes of methanol and filtered before the HPLC measurement. Samples were prepared in 2 ml vials, capped and automatically injected in the HPLC column at a draw speed of 350 micro-l/min, with 20 micro-l injection volume, at 1.2 ml/min flow rate and with detector wavelength at 276 nm. A calibration curve was prepared based on purified frenolicin compounds. The retention times for frenolicin, deoxyfrenolicin and frenolicin B are 5.7, 8.1 and 11.6 minutes, and the detector response factors (g/l product concentration/peak area) are 3.40 x 10E-8, 8.47 x 10E-9 and 9.28 x 10E-9, respectively.

### EXAMPLE 1

*Streptomyces roseofulvus* mutant strain, deposited with the American Type Culture Colection, Maryland, U.S.A. as ATCC No. 55598, was used as the microorganism for fermentation. The spore stock, 5 ml aliquots frozen at -70°C with 5% lactose and 10% glycerol, were thawed, serially diluted and spread on agar plates containing 0.5 g/l of yeast extract (Amberex 1003, Red Star®, Universal Foods), 0.5 g/l of asparagine (Sigma), 39 g/l of potato dextrose agar (Difco) and 10 g/l of agar (Difco), in deionized water to grow spores in the incubator at 27°C for 7 days. The spores were scraped off from the plates using 5 g/l of sterile agar solution. About 10⁸ spores were used to inoculate a 1 l seed medium in 6 l Erlenmeyer flasks. The seed medium contained, per 1, 5 g of yeast extract (Amberex 1003, Red Star®, Universal Foods), 5 g of Bacto tryptone (Difco), 10 g of glucose, 20 g of corn starch (Eclipse® N, Staley) and 4 g of calcium carbonate (Whittaker Clark Daniels) in deionized water. The flasks were incubated at 27°C in a rotary shaker operating at 220 revolutions per minute (rpm) for 48 hours. 0.8 l of the grown microorganisms in the seed medium was used to inoculate a 40 l fermentation batch. 60 l of seed inoculum was used to inoculate a 1,325 l working volume of fermentation broth in a 2,000 l fermentor. The broth (1,325 l) contained initially, per litre, 50 g of corn oil (Welch, Holme and Clark), 20 g of dextrin (Sigma type IV), 15 g of corn steep solids (Roquette), 10 g of cane molasses (Mid-Eastern), 7.5 g of sodium formate (Fisher), 3 g of yeast extract (Amberex 1003), 4 g of magnesium sulphate heptahydrate (Fisher), 0.44 g of monosodium phosphate heptahydrate (Fisher), 4 g of calcium carbonate (Pfizer) and 0.3 ml of an antifoam agent (Union Carbide, UCON® LB-625, polypropylene glycol monobutyl ether).

The fermentation was conducted at 27°C, 5 psig back pressure, dissolved oxygen maintained above 30% by controlling the agitation between 160 and 280 rpm, and by controlling the aeration between 336 and 672 l/min. The pH of the fermentation broth was not controlled and measured between 6.5 and 8.4.

After the completion of fermentation (186 hours, 1,325 l, pH 8.2, frenolicin 3.8 g/l, deoxyfrenolicin 0.1 g/l), the aeration in the fermentor (2,000 l) was turned off and the fermentation broth was stripped of oxygen by passage of nitrogen. The fermentor agitation was reduced from 280 to 100 rpm.

The fermentation broth temperature was reduced from 27°C to 10°C with chilled water and held for 7 hours to convert frenolicin to deoxyfrenolicin. The converted broth (1,325 l, pH 7.7, frenolicin 0.2 g/l, deoxyfrenolicin 2.9 g/l) was adjusted to pH 11.5 using 12.9 l of 50% aqueous sodium hydroxide and mixed for 30 minutes under nitrogen. The broth pH was then adjusted to 2.5 using 14 l of concentrated sulphuric acid and immediately extracted with ethyl acetate (1,325 l) for 30 minutes.

The whole fermentation broth and ethyl acetate extract mixture was centrifuged in a Westfalia disk type separator (Model SA-7-06-076) at 280 l/h flow rate. The separated ethyl acetate extract (light phase, 1,298 l, pH 2.7, frenolicin 0.12 g/l, deoxyfrenolicin 2.91 g/l) was concentrated in a wiped-film evaporator (Artisan, BD410, EVP-122) at 50°C, 28" vacuum (absolute pressure of 1.9 inches/48.3 mm of mercury) and 52 l/h feed flow rate. The ethyl acetate extract concentrate (190 l, pH 3.0, deoxyfrenolicin 19.5 g/l, frenolicin 0.83 g/l) was washed with 93 l of deionized water, adjusted to pH 6.1 with concentrated ammonium hydroxide and mixed for 30 minutes. The washed ethyl acetate concentrate was separated by phase separation, mixed with 32 l of deionized water and adjusted to pH 3 with concentrated hydrochloric acid.

The separated and water-washed ethyl acetate extract concentrate layer (224 l, pH 3, deoxyfrenolicin 16.3 g/l) was further concentrated in a rotary evaporator (50 l, 30 l capacity, Buchi Rotavapor Model 185 Ex, EVP-454) at 40°C and 28" vacuum to an oily paste. The oily paste (28 l) was extracted with 430 l of methanol. The separated upper phase methanol extract (426 l, pH 3.2, deoxyfrenolicin 8.28 g/l) was adjusted to pH 6.1 with 1M ammonium hydroxide. The pH-adjusted methanol extract was warmed (reacted) at 38°C under 10 psig air for 12 hours to convert deoxyfrenolicin to frenolicin B. The reaction was quenched by adjusting the methanol extract to pH 3.0 with 1M hydrochloric acid.

The quenched methanol extract (426 l, frenolicin B 7.35 g/l, deoxyfrenolicin 0.2 g/l) was concentrated in the wiped-film evaporator at 45°C, 28" vacuum and 20 l/h feed flow rate. The concentrated methanol extract slurry (66 l, frenolicin B 46.4 g/l) was cooled at 0°C overnight to crystallize frenolicin B and filtered (Whatman No. 1 filter). The wet frenolicin B cake was washed with cold acidified methanol (pH 2) and vacuum (25"; equivalent to absolute pressure of 4.9 inches/124.5 mm of mercury) dried at 30°C overnight. The dried crude frenolicin B solids were reslurried in n-hexane (37 l) to remove oils and filtered. The hexane-washed solids were vacuum-dried to obtain 3,060 g of frenolicin B with 90% purity.

The 90% purity frenolicin B was further purified by dissolving the frenolicin B solids in ethyl acetate to a concentration of 80 g/l. The frenolicin B ethyl acetate solution was filtered (Whatman No. 1 filter) and concentrated under nitrogen and 25" vacuum at 35°C in a crystallizer to a frenolicin B concentration of 300 g/l. The concentrate was cooled at a programmed rate of 2.1°C/h for 16 hours to 0°C. The crystallized frenolicin B was filtered, washed with cold (4°C) 50/50 (v/v)% ethyl acetate and n-hexane, and dried under vacuum to obtain 2,640 g of frenolicin B with 98.5% purity.

### EXAMPLE 2

The *Streptomyces roseofulvus* seed inoculum preparation, mycelial fermentation, frenolicin conversion to deoxyfrenolicin in the fermentation broth, whole broth extraction with ethyl acetate, centrifugation and ethyl acetate extract concentration procedures are the same as those described in Example 1. The ethyl acetate extract concentrate (26 l, deoxyfrenolicin 19.8 g/l, frenolicin 1.4 g/l) was further concentrated in a rotary evaporator at 40°C and 28" vacuum (absolute pressure of 1.9 inches/48.3 mm of mercury) to distill off residual ethyl acetate to an oily paste. To the oily paste (12 l, deoxyfrenolicin 42 g/l) were added 18 l of n-hexane, and the whole was mixed for 0.5 hours to dissolve the oils. The deoxyfrenolicin hexane slurry was filtered under 25" vacuum (absolute pressure of 4.9 inches/124.5 mm of mercury) with Whatman filter paper. The crude deoxyfrenolicin cake (816 g, 56% purity) was washed with 3 l of n-hexane and dried in a vacuum (25") oven at 30°C overnight. The combined filtrate and hexane wash (32 l, deoxyfrenolicin 1.4 g/l) can be concentrated and recycled.

The filtered and dried crude deoxyfrenolicin solids (816 g, 56% purity) were redissolved in ethyl acetate (10 l) to a deoxyfrenolicin concentration of 45 g/l. The pH of the deoxyfrenolicin in ethyl acetate solution was adjusted to pH 6.1 with ammonium hydroxide. The solution was heated to 45°C with mixing and ethyl acetate reflux, and the mixing vessel was blanketed with air at 5 to 10 psig. The pH of solution during the reaction was maintained at 5.9 to 6.1 with addition of ammonium hydroxide. Deoxyfrenolicin was converted to frenolicin B with 92% conversion in 24 hours. The converted frenolicin B in ethyl acetate solution (9.5 l, frenolicin B 43 g/l) was concentrated in the rotary evaporator at 40°C and 28" vacuum to evaporate ethyl acetate. The solvent-free crude frenolicin B solid slurry (1.2 l) was mixed with 2.4 l of n-hexane and filtered under 25" vacuum with Whatman filter paper, washed with n-hexane and vacuum (25") oven dried at 30°C overnight. The frenolicin B solid (445 g, 87 % purity) can be further purified with the same recrystallization procedures as those described in Example 1.

## Claims

1. A process for producing deoxyfrenolicin, and if desired, frenolicin B, which process is characterized by a) fermenting a broth containing a microorganism capable of producing frenolicin under aerobic conditions to produce frenolicin, then b) converting the frenolicin in the same broth under anaerobic conditions to deoxyfrenolicin, and if desired, c) converting the deoxyfrenolicin to frenolicin B during the subsequent recovery and purification steps.

2. A process according to claim 1, wherein the microorganism is a mutant strain of *Streptomyces roseofulvus*.

3. A process according to claim 1 or 2, wherein the microorganism is the mutant strain of *Streptomyces roseofulvus* deposited with the American Type Culture Collection under the ATCC No. 55598.

4. A process according to any one of claims 1 to 3, wherein the step b) comprises stripping the broth of oxygen with nitrogen from the point in time when the titer of frenolicin is at or approximately at its maximum.

5. A process according to claim 4, wherein the step b) further comprises holding the broth under anaerobic conditions for about 2 to about 8 hours at a pH of about 7.0 to about 9.0.

6. A process according to claim 5, wherein the pH is about 7.5 to about 8.4.

7. A process according to any one of claims 4 to 6, wherein after establishment of completion of the conversion, the pH of the broth is adjusted to about 10 to about 11.5 by addition of an appropriate amount of a base and held for an additional 0.5 to 1 hour under anaerobic conditions.

8. A process according to any one of claims 1 to 7, wherein the step c) involves warming the deoxyfrenolicin to a temperature up to about 45°C.

9. A process according to claim 8, wherein the warming is effected at an excess pressure above atmospheric pressure of about 0.35 to about 0.70 kg/cm².
